# EUROPEAN PATENT SPECIFICATION

(11) **EP 2 654 806 B1**
(45) Date of publication and mention of the grant of the patent: **22.05.2019**
(21) Application number: 11804697.8
(22) Date of filing: 22.12.2011
(51) Int. Cl.: A61L 2/08

(54) **PHOTODYNAMIC CONTROL OF MICROBIAL GROWTH ON SURFACES**
FOTODYNAMISCHE STEUERUNG VON MIKROBIELLEM WACHSTUM AUF OBERFLÄCHEN
CONTRÔLE PHOTODYNAMIQUE DE LA CROISSANCE MICROBIENNE SUR LES SURFACES

(30) Priority: 22.12.2010 EP 10196674; 18.04.2011 EP 11162850
(43) Date of publication of application: 30.10.2013
(73) Proprietor: Ortner Cleanroom Engineering GmbH, 9500 Villach (AT)
(72) Inventor: BERG, Gabriele, A-8010 Graz (AT); LIEBMINGER, Stefan, A-8041 Graz (AT); OBERAUNER, Lisa, A-8010 Graz (AT); KLEIN, Thomas, A-8652 Kindberg (AT); STAMPF, Roland, A-9500 Villach (AT)
(74) Representative: Dilg, Andreas
(86) International application number: PCT/EP2011/073893
(87) International publication number: WO 2012/085257

(56) References cited:
- WO-A1-99/49823
- WO-A1-2005/054217
- FR-A1- 2 924 445
- US-A1- 2006 223 729
- US-B1- 6 420 455
- WAINWRIGHT M ET AL: "Phenothiazinium-based photobactericidal materials", JOURNAL OF PHOTOCHEMISTRY AND PHOTOBIOLOGY B: BIOLOGY, ELSEVIER SCIENCE S.A., BASEL, CH, vol. 84, no. 3, 1 September 2006 (2006-09-01), pages 227-230, XP025227644, ISSN: 1011-1344, DOI: 10.1016/J.JPHOTOBIOL.2006.03.002 [retrieved on 2006-09-01]

## Description

### FIELD OF THE INVENTION

The present invention relates to methods for controlling microbial growth on a surface to be treated, particularly on clean room clothing. More particularly, the invention is directed to methods for the decontamination of such surfaces by providing photosensitizers that exhibit antimicrobial efficacy upon activation by electromagnetic radiation and exposing the surfaces to such electromagnetic radiation.

### BACKGROUND

Clean room environments are of immense value in the fabrication and assembly of many industrial products, including *inter alia* electronics, semiconductors, spacecraft components, medical devices, pharmaceuticals, and cosmetics. The low density of environmental pollutants such as dust, aerosolized particulates, airborne microorganisms, and chemical vapors within these clean room facilities reduces the amount of both inorganic and organic (i.e. biological) contaminations on and within the products manufactured.

Typically, a clean room has a controlled level of contamination that is specified by the number of particles per cubic meter at a specified particle size (0.1 or 0.5 µm in diameter). Clean rooms are classified according to, e.g., the EN ISO 14644-1 standard (class ISO 1 to ISO 9) or the EU GMP Annex 1 standard for sterile pharmaceuticals (class A to D). In a clean room of class A/B according to EU GMP Annex 1 standard (corresponding to ISO 5) the maximally allowed number of particles (0.5 µm in diameter) amounts to about 3500, in a clean room of class C (corresponding to ISO 7) to about 3.5 x 10⁵, and in a clean room of class D (corresponding to ISO 8) to about 3.5 x 10⁶, respectively.

The maintenance of a clean environment and the prevention (or at least reduction) of further contamination is essential for the production process of the above-referenced goods. For example, in the pharmaceutical industry an aseptic environment (at least during some production steps) is inevitable for the manufacture of medicaments (cf., e.g., Schicht, H.H. (1998) Cleanrooms Int. 2, 17-28).

Clean room technology thus aims at the development of various concepts for avoiding contamination. For example, equipment inside the clean room such as furniture is designed to generate minimal air contamination. Air entering a clean room from outside is filtered to exclude dust, and the air inside is constantly re-circulated through high efficiency particulate air filters in order to remove internally generated contaminants. In addition, clean rooms may also be kept at a positive pressure so that in case of any leaks, air leaks out of the chamber instead of unfiltered air coming in.

One critical source of contamination is the transfer of materials or personnel to or from clean rooms. However, some equipment, typically made of materials having a low porosity, such as manufacturing devices (e.g. pipetting robots) or laboratory supplies (e.g., tubes, dishes) can be decontaminated (or sterilized) fairly easy by means of, e.g., autoclaving (that is, heat treatment) or gamma ray treatment.

Much more problematic is the transfer of people to, from or between different clean rooms. Personnel enter and leave clean rooms through airlocks (sometimes including an air shower stage), and wear protective clothing such as masks, gloves, shoe covers, and coveralls. However, whenever staff members move between clean rooms of different classes (e.g., for monitoring in parallel different production (and/or packaging) steps or to transport an intermediate product obtained in a given production step in another clean room where the next step takes place) have to change their complete protective clean room clothing in order to avoid microbial contaminations. This procedure is not only uncomfortable for the staff concerned but also time consuming (about 30 min per change of clothing). Since, in average, there are 3-4 coating cycles per day, the total expenditure of time can be calculated to be about 2.5 hours per day.

In addition, after each coating cycle the clean room clothing has to be disposed (in case of single use-clothing) or washed and sterilized prior to re-use. Employing single use-clothing is clearly an expensive option. Re-using the clean room clothing, on the other hand, requires considerable effort. Moreover, sterilization proceedings by heat treatment or radiation affect, at least in the medium-term, the quality and performance characteristics of the clothing material such that the re-use of such protective clothing is also limited to a clear number of cycles (cf., e.g., Gail, L. and Hortig, H.P. (2004) Reinraumtechnik, 2nd ed., Springer Verlag, Berlin, p. 319-354).

Hence, there is a continuing need for clean room clothing having improved functional properties that would not necessarily have to be changed during each and every entry in or leave of clean rooms. In order to reduce microbial contaminations clean room clothing with antimicrobial activity has previously become available. For example, the antimicrobial effect can be accomplished by incorporation of chemical compounds such as gold and/or silver fibers into the clothing material. However, the supposed advantages of this approach are still controversially discussed, particularly in view of the significantly higher costs of such equipment. Furthermore, chemical agents often show a highly selective antimicrobial activity that affects only a particular portion of microbial contaminants present in an environment.

On the other hand, there is also burgeoning evidence that clean rooms are colonized by populations of specialized and highly adapted microorganisms that are capable of tolerating such extreme environmental conditions, in particular the limited nutrition supply (cf., e.g., La Duc, M.T. et al. (2007) Appl. Environ. Microbiol. 73, 2600-2611). The known and rather general inhibitory effects of, e.g., gold and silver on microbial growth may thus likely be insufficient to decontaminate clean room equipment colonized with such extremo-tolerant microorganisms.

Thus, there still remains a need for methods and compositions for controlling microbial growth on clean room equipment, particularly clean room clothing, enabling a reliable and efficient treatment even of contaminations caused by extremo-tolerant microorganism. In particular, there is a need for corresponding means allowing for the decontamination of clean room clothing in order to avoid the change of the protective clothing at each and every entry in or leave of a clean room.

The removal of microbial contaminants is also of utmost importance in clinical settings. Common hospital surfaces such as room door handles, sterile packaging, ward fabrics and plastics, stethoscopes, and the like are frequently contaminated by potentially harmful (i.e. pathogenic) microorganisms that may cause detrimental health risks. Such surfaces may act as reservoirs of microorganisms which could lead to the spread of contamination upon being touched, by either healthcare workers or patients.

In addition to this, there is mounting evidence of a link between contaminated surfaces and nosocomial (i.e. hospital-acquired) infections (cf., e.g., Talon, D. (1999) J. Hospital Infect. 43, 13-17; Bhalla, A. et al. (2004) Infect. Contr. Hospital Epidemiol. 25, 16-167). Nosocomial infections can often lead to severe complications with existing illnesses and cause or contribute to about 100.000 deaths each year in the USA and about 50.000 deaths each year in Europe. Combining knowledge of pathogen survival on surfaces, and the evidence for transmission of pathogens from surfaces to hands, the importance of the inanimate hospital environment is immediately evident.

The problem is not only that reservoirs of potentially pathogenic microorganisms exist but also that they can persist for long periods due to the ability of some microorganisms to survive on surfaces for days, weeks, and even months. It is widely recognized that the usual means of attempting to reduce the microbial load on environmental surfaces are ineffective. Detergent solutions, for example, often become contaminated during use, thus allowing the spread of microbes throughout the hospital via mops and cleaning cloths.

Furthermore, in clinical settings the decontamination of surfaces is additionally hampered by the often high concentration of multidrug-resistant pathogenic microorganisms present in such environments, that is, microbial contaminants that have acquired resistance against various drugs or other chemical compounds (in particular antibiotics), which would otherwise exert a growth inhibitory and/or killing anti-microbial effect.

Therefore, there is also a continuous need to develop new approaches to reducing microbial contamination of the hospital environment. In particular, highly potent antimicrobial agents having a broad range activity are required that enable their use for the removal even of multidrug-resistant pathogens without the risk that the contaminants acquire resistance against the antimicrobial agents employed.

US 6,420,455 B1 discloses antimicrobial compositions containing photosensitizers articles, and methods of use. In particular, polymer compositions and articles incorporating them are described that possess antimicrobial activity, preferably in both the light and the dark. Such compositions include one or more polymers and one or more xanthene photosensitizers. FR 2 924 445 A1 discloses textile fibers with disinfectant properties by means of anthraquinone derivatives. WO 99/49823 discloses antimicrobial plastic material containing an agent such as methylene blue dispersed therein which, upon exposure to light, generates singlet oxygen having antimicrobial activity. Wainwright M. et al. (2006) Journal of Photochemistry and Photobiology B: Biology 84, 227-230 discloses phenothiazinium-based photobactericidal materials. WO 2005/054217 A1 discloses phenothiazinium compounds suitable as antimicrobial agents. US 2006/0223729 A1 discloses methods of inactivating bacterial spores wherein the bacterial spores are contacted with a photosensitizer and irradiated such that a phototoxic species is produced that inactivates the bacterial spores.

### SUMMARY OF THE INVENTION

The invention is defined by the appended independent claims. Any information falling outside the scope of the claims is for explanation only. In a first aspect, the present invention relates to a method for controlling microbial growth on a surface to be treated, the method comprising: exposing the surface to be treated to electromagnetic radiation, the electromagnetic radiation being emitted from one or more electromagnetic radiation sources towards the surface, wherein one or more photosensitizers being activatable by electromagnetic radiation are covalently attached to the surface to be treated, the electromagnetic radiation being emitted has a wavelength in a range between 400 nm and 800 nm, the one or more photosensitizers exhibit antimicrobial efficacy upon activation by electromagnetic radiation; the surface to be treated is part of clean room clothing and the method is performed after having put on the clean room clothing to be treated, wherein the surface is exposed to electromagnetic radiation for a period of time being less than 5 min and with a radiation dosage between 1 J/cm² and 200 J/cm².

In preferred embodiments, the electromagnetic radiation being emitted has a wavelength in a range between 580 nm and 650 nm, and particularly in a range between 610 nm and 640 nm.

In further preferred embodiments, the one or more photosensitizers are reactive dyes, and particularly reactive dyes being selected from the group consisting of methylene blue, methyl methylene blue, dimethyl methylene blue, new methylene blue, toluidine blue, Rose Bengal, acridine orange, hypericin, and combinations thereof. Particularly preferably, the one or more photosensitizers are selected from the group consisting of methyl methylene blue, dimethyl methylene blue, new methylene blue, and combinations thereof.

In particularly preferred embodiments, the surface to be treated is a textile fabric, and particularly a textile fabric comprising fibers being selected from the group consisting of polyester fibers, cellulose acetate fibers, and combinations thereof.

In specific embodiments, the surface is exposed to electromagnetic radiation for a period of time being less than 3 min, and particularly less than 1 min.

In particularly preferred embodiments, the one or more electromagnetic radiation sources are light emitting diodes.

In specific embodiments, the method is for controlling growth of clean room associated microorganisms, particularly of any one or more clean room associated microorganisms being selected from the group consisting of *Bacillus spec*., *Paenibacillus spec*., *Geobacillus spec*., *Oceanobacillus spec*., *Micrococcus spec*., *Staphylococcus spec*., *Exiguobacterium spec*., *Microbacterium spec*., *Kocuria spec*., *Pseudomonas spec*., *Sphingomonas spec*., *Stenotrophomonas spec*., *Verticillium spec*., *Penicillium spec*., *Candida spec*., and *Saccharomyces spec*..

In further specific embodiments, the method is for controlling growth of human pathogens, particularly of multidrug-resistant human pathogens, and more particularly of any one or more microorganisms being selected from the group consisting of *Staphylococcus aureus*, *Staphylococcus epidermidis*, *Pseudomonas aeruginosa*, *Haemophilus influenzae*, *Stenotrophomonas maltophilia*, *Acinetobacter baumanii*, *Clostridium difficile*, *Mycobacterium tuberculosis*, and *Legionella pneumophila.*

In a second aspect, the use of a method, as defined herein, for the microbial decontamination of a surface to be treated is described.

In a third aspect, which no longer forms part of the invention, an arrangement for controlling microbial growth on a surface to be treated is described, the arrangement comprising: a surface provided with one or more photosensitizers being activatable by electromagnetic radiation, one or more electromagnetic radiation sources being arranged to emit electromagnetic radiation towards the surface to be treated wherein the one or more electromagnetic radiation sources emit electromagnetic radiation having a wavelength in a range configured for activating the one or more photosensitizers, the electromagnetic radiation being emitted has a wavelength in a range between 400 nm and 800 nm, and wherein the one or more photosensitizers are capable of exhibiting antimicrobial efficacy upon activation.

In preferred embodiments of the arrangement, the surface to be treated is a clean room suit, comprising a fabric, and particularly a fabric comprising fibers being selected from the group consisting of polyester fibers, cellulose acetate fibers, and combinations thereof.

In further preferred embodiments of the arrangement, the one or more photosensitizers are reactive dyes, and particularly reactive dyes being selected from the group consisting of methylene blue, methyl methylene blue, dimethyl methylene blue, new methylene blue toluidine blue, Rose bengal, acridine orange, hypericin, and combinations thereof. Particularly preferably, the one or more photosensitizers are selected from the group consisting of methyl methylene blue, dimethyl methylene blue, new methylene blue, and combinations thereof.

In specific embodiments, the one or more photosensitizers are covalently attached to the surface to be treated.

In further specific embodiments, the arrangement further comprises a clean room lock for cleaning a person transmitting between an interior and an exterior of a clean room, wherein the clean room suit is for clothing a person in the clean room lock.

In particularly preferred embodiments of the arrangement, the one or more electromagnetic radiation sources are light emitting diodes.

In specific embodiments, the light emitting diodes are integrated in one or more walls of the clean room lock.

In a forth aspect, which no longer forms part of the invention, a clean room comprising an arrangement, as defined herein is described.

Finally, in a fifth aspect, which no longer forms part of the invention, the use of an arrangement, as defined herein, or a clean room, as defined herein, for the microbial decontamination of a surface to be treated is described.

### DESCRIPTION OF THE DRAWINGS

**FIGURE 1****: Photosensitizers according to the present invention.**
   **(A)** Schematic representation of the chemical structures of five exemplary reactive dyes that can be employed as photosensitizers according to the invention. **(B)** Spectral analysis of methylene blue revealing two absorption maxima at 610 nm and 664 nm, respectively.
**FIGURE 2****: Photosensitizers according to the present invention.**
   Schematic representation of the chemical structures of methylene blue and seven exemplary derivatives thereof (including toluidine blue) that can be employed as photosensitizers according to the invention (adapted from Wainwright, M. and Amaral, L. (2005) Trop. Med. Int. Health 10, 501-511). Another exemplary derivative, methyl methylene blue, has an identical structure as methylene blue except for a methyl group at substituent R¹.

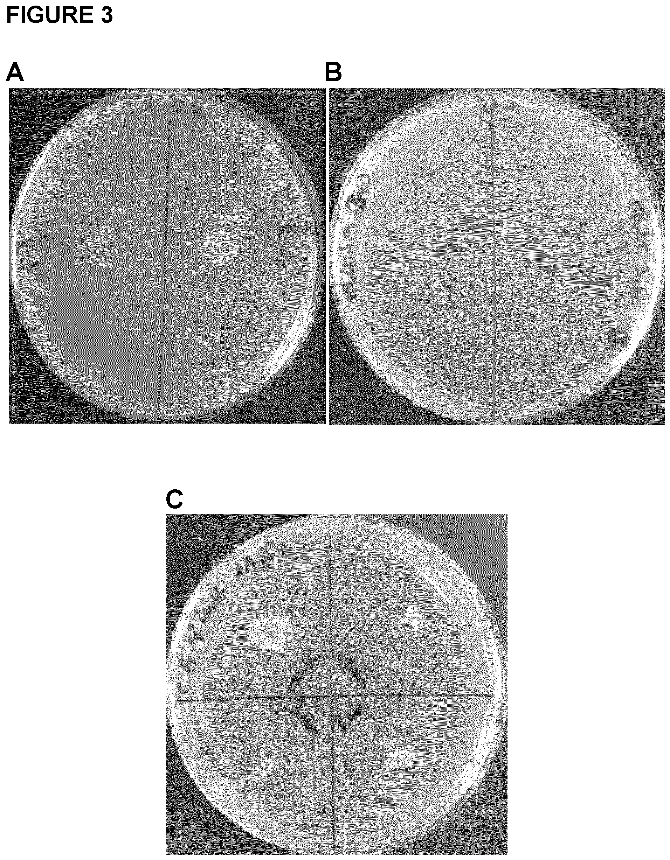

**FIGURE 3****: Analysis of the antimicrobial efficacy of methylene blue attached to textile fabrics (contact assay).**
   Pieces of a textile fabric (ION-NOSTAT VI.2; Dastex Reinraumzubehör, Muggensturm, Germany) having a defined size (1 x 1 cm) were immersed for 3 h in an aqueous solution of methylene blue (100 µg/ml). After complete drying of the textile pieces, 3 µl of a growing cell culture (titer: 7 x 10⁷ cfu/ml) of *Staphylococcus aureus*, *Stenotrophomonas maltophilia*, and *Candida albicans*, respectively, were added to the surface of the textile pieces. The textile pieces were contacted with agar plates; and the plates were incubated at 37 °C for 24 h. **(A)** Control plate; contact sampling of textile pieces without further treatment: S. *aureus* (left), S. *maltophilia* (right). **(B)** Test plate; contact sampling of textile pieces after irradiation for 3 min with a laser (15 mW) at a wavelength of 532 nm: *S*. *aureus* (left), *S*. *maltophilia* (right). **(C)** Corresponding analysis for *C. albicans;* shown are (clockwise, starting at top left) the control, and the test samples after irradiation for 1 min, 2 min, and 3 min, respectively.

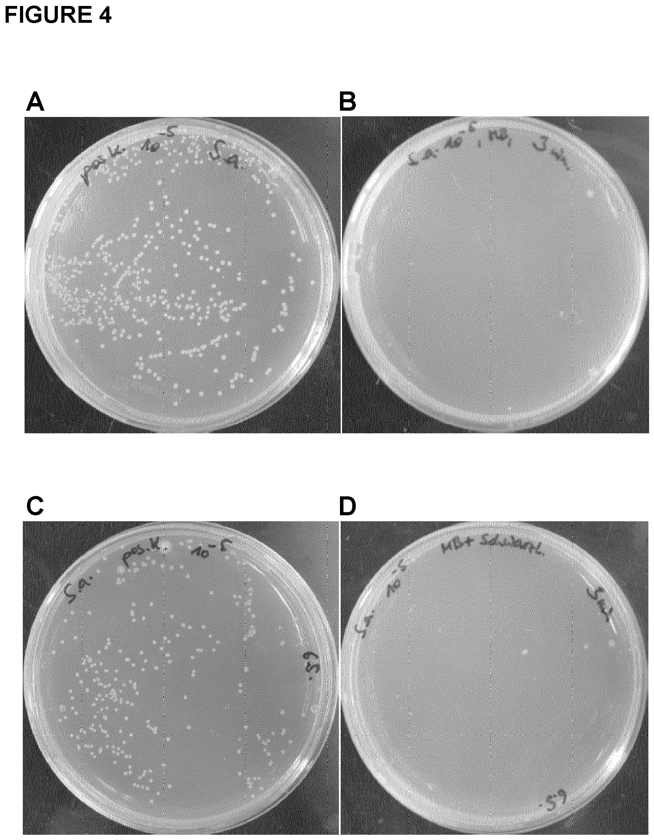

**FIGURE 4****: Analysis of the antimicrobial efficacy of methylene blue (plating assay).**
   In a well of a microtiter plate, 50 µl of a growing cell culture (titer: 7 x 10⁷ cfu/ml) of *Staphylococcus aureus* (*S. aureus*) were mixed with aqueous solution of methylene blue (100 µg/ml). **(A)** Control sample; directly plated on an agar plate without further treatment. **(B)** Test sample after irradiation for 3 min with a mercury lamp at a wavelength of 515-560 nm. **(C)** Control sample; directly plated on an agar plate without further treatment. **(D)** Test sample after irradiation for 3 min with a black light lamp at a wavelength of 350-370 nm. The plates were incubated at 37 °C for 24 h.

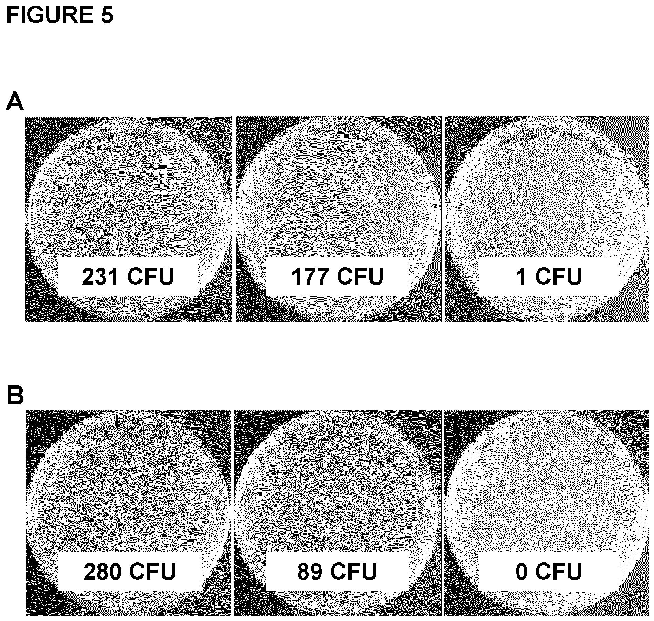

**FIGURE 5****: Analysis of the antimicrobial efficacy of methylene blue and toluidine blue (plating assay).**
   The assay was performed as described above in the context of Fig. 4 using aqueous solutions of **(A)** methylene blue or **(B)** toluidine blue, respectively (100 µg/ml each). *S*. *aureus* was employed as test organism. The following conditions were used: cell culture, no incubation with dye, no irradiation (left); cell culture, incubation with dye, no irradiation (middle); cell culture, incubation with dye, irradiation for 3 min with a mercury lamp at a wavelength of 515-560 nm (right). The respective numbers of colonies (colony forming units; cfu) obtained after incubation are indicated.

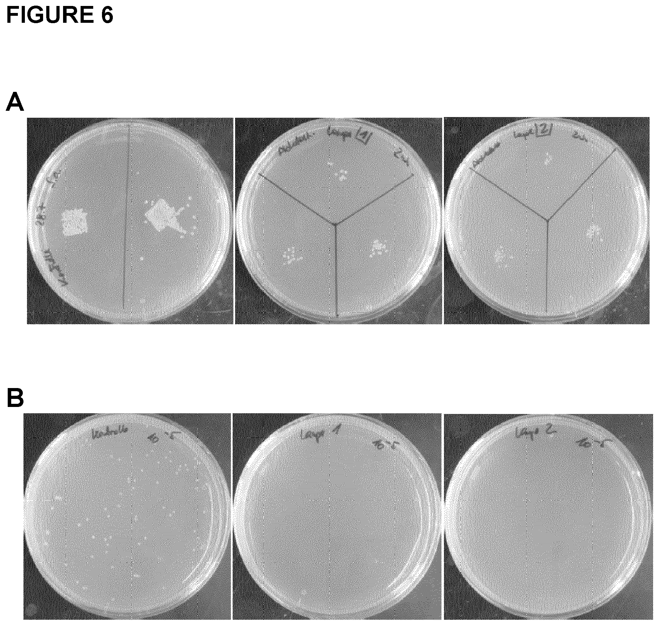

**FIGURE 6****: Analysis of the antimicrobial efficacy of methylene blue at a wavelength of 620-640 nm (contact and plating assays).**
   The contact **(A)** and plating **(B)** assays were performed as described above in the context of Fig. 3 and Fig. 4, respectively, using an aqueous solution of methylene blue (100 µg/ml). *S*. *aureus* was employed as test organism. Irradiation was accomplished by means of LEDs at a wavelength of 620-640 nm. LED1 used as illuminant 38 GU5.3 highpower, LED2 used as illuminant MR16 (both from Conrad, Hirschau, Germany). The following conditions were used in **(A):** textile piece, incubation with dye, addition of cell culture, no irradiation (left); textile piece, incubation with dye, addition of cell culture, irradiation for 2 min with LED1 (middle); textile piece, incubation with dye, addition of cell culture, irradiation for 2 min with LED2 (right). The following conditions were used in **(B):** cell culture, no incubation with dye, no irradiation (left); cell culture, incubation with dye, irradiation for 2 min with LED1 (middle); cell culture, incubation with dye, irradiation for 2 min with LED2 (right).

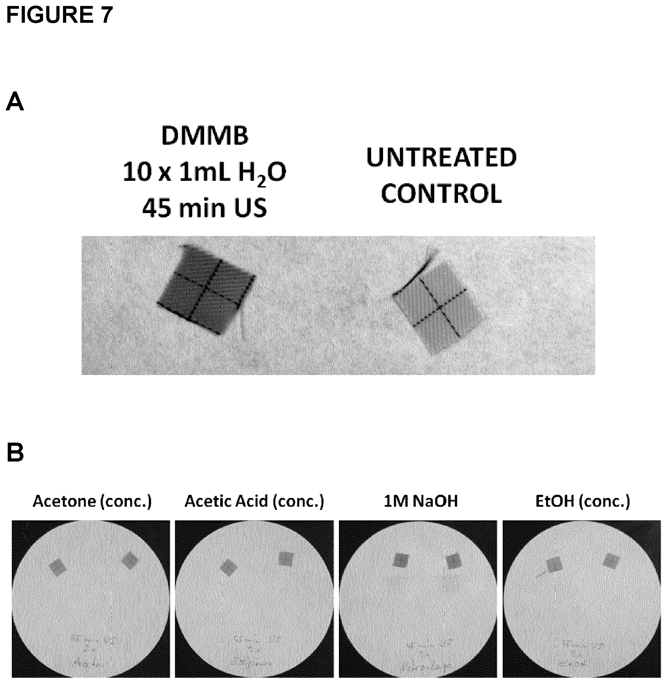

**FIGURE 7****: Staining of textile fabrics with dimethyl methylene blue.**
   **(A)** Pieces of a textile fabric (ION-NOSTAT VI.2; Dastex Reinraumzubehör, Muggensturm, Germany) having a defined size (1 x 1 cm) were stained with 100 mg/l dimethyl methylene blue for 15 min in an autoclave (121 °C, 210 kPa). The pieces were repeatedly (at least five times) washed in distilled water to remove excess dye, and then subjected to an ultrasound treatment for 45 min in order to remove unbound dye not attached to the fabric. An exemplary textile piece is shown at the left. An unstained control is shown at the right. **(B)** Analysis of the resistance to chemicals. Stained pieces of textile fabric were repeatedly (at least three times) immersed in the following chemicals (shown from left to right): 99% (v/v) acetone, 99% (v/v) acetic acid, 1 M sodium hydroxide (NaOH), and 99% (v/v) ethanol (EtOH). The wet pieces were then placed on filter paper, and the extent of dye released from the pieces was determined. No dye was released after two cycles of immersion.

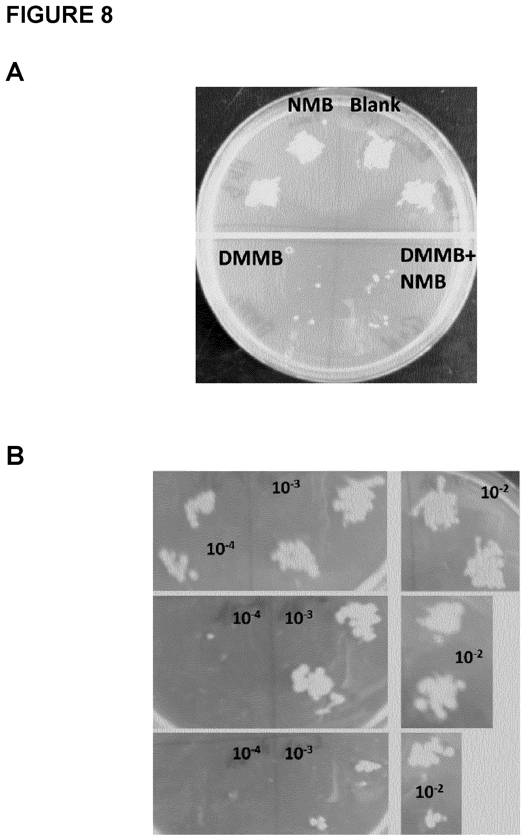

**FIGURE 8****: Analysis of the antimicrobial efficacy of dimethyl methylene blue and new methylene blue (contact assay).**
   The assay was performed as described above in the context of Fig. 3 using an aqueous solution of diemethyl methylene blue (DMMB) and new methylene blue (NMB), respectively (100 µg/ml each). *Staphylococcus aureus* **(A)** as well as *Klebsiella pneumoniae* **(B)** were employed as test organisms. Irradiation was accomplished by means of a LED (High Power Alustar 3 W 10° LEDxON Rot, from Conrad, Hirschau, Germany) at a wavelength of 623 nm. The following conditions were used in **(A):** textile piece, no incubation with dye, no irradiation (right top); textile piece, incubation with NMB, addition of cell culture (about 10⁴ cfu), irradiation for 1 min (left top); textile piece, incubation with DMMB, addition of cell culture, irradiation for 1 min (left bottom); textile piece, incubation with NMB + DMMB, addition of cell culture, irradiation for 1 min (right bottom). The following conditions were used in **(B):** textile piece, incubation with DMMB, addition of cell culture (about 10⁴ cfu), irradiation for 2 min (top), 5 min (middle), and 10 min (bottom), respectively. In each experiment, three different dilutions of the cell cultures (10⁻², 10⁻³, and 10⁻⁴) were analyzed.

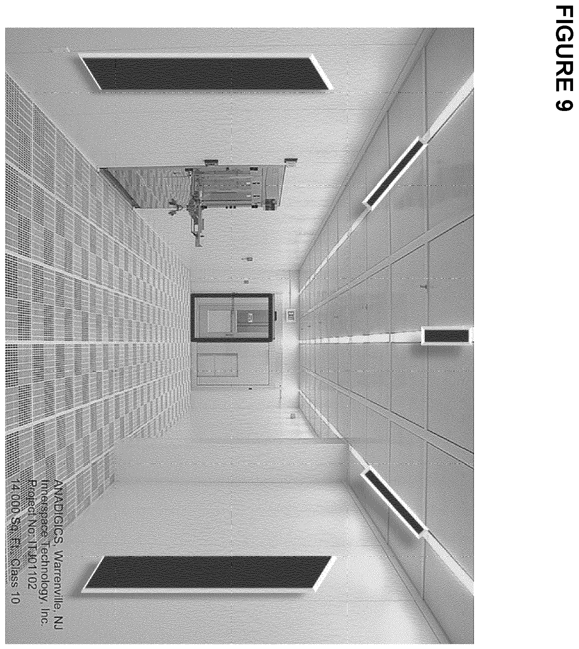

**FIGURE 9****: Clean room adapted for practicing the present invention.**
   Shown is an exemplary embodiment of a clean room that is adapted for practicing the present invention. The clean room is equipped with six electromagnetic radiation sources (shown as dark gray areas): three being arranged at the ceiling, two at the left and right side walls, and one surrounding the entrance in the back. The radiation sources may be used for permanently irradiating the interior of the clean room.

### DETAILED DESCRIPTION OF THE INVENTION

The present invention is based on the unexpected finding that the provision of a surface to be decontaminated with one or more photosensitizers being activatable by electromagnetic radiation and exposure of the surface to electromagnetic radiation of appropriate wavelength for activating the photosensitizers represents a successful tool for (biologically) controlling microbial growth on such surface, in particular as the use of harmful UV radiation is typically avoided. This, in turn, enables for a reliably and efficient decontamination of such surfaces even in extreme environments (e.g., in clean rooms or on clean room clothing) or in clinical settings, frequently requiring the inhibition and/or killing of human pathogens, without the risk that the contaminants acquire resistance against the antimicrobial agents employed. The latter is particularly accomplished by the non-selective mode of action of the photosensitizers being employed. Typically, upon radiation-induced activation such photosensitizers produce reactive chemical species (e.g., singlet oxygen) exerting antimicrobial efficacy.

The methods and compositions described herein also enable a reliably and efficient decontamination of clean room clothing, thus facilitating clean room operations by avoiding the otherwise necessary change of the protective clothing at each and every entry in or leave of a clean room.

The present invention will be described in the following with respect to particular embodiments and with reference to certain drawings but the invention is to be understood as not limited thereto but only by the appended claims. The drawings described are only schematic and are to be considered non-limiting.

Where the term "comprising" is used in the present description and claims, it does not exclude other elements or steps. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Where an indefinite or definite article is used when referring to a singular noun e.g. "a", "an" or "the", this includes a plural of that noun unless specifically stated otherwise.

In case, numerical values are indicated in the context of the present invention the skilled person will understand that the technical effect of the feature in question is ensured within an interval of accuracy, which typically encompasses a deviation of the numerical value given of ± 10%, and preferably of ± 5%.

Furthermore, the terms first, second, third, (a), (b), (c), and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context of which the terms are used. The following terms or definitions are provided solely to aid in the understanding of the invention. These definitions should not be construed to have a scope less than understood by a person of ordinary skill in the art.

In a first aspect, the present invention relates to a method for controlling microbial growth on a surface to be treated, comprising: exposing the surface to be treated to electromagnetic radiation, the electromagnetic radiation being emitted from one or more electromagnetic radiation sources towards the surface, wherein:
- one or more photosensitizers being activatable by electromagnetic radiation are covalently attached to the surface to be treated;
- the electromagnetic radiation being emitted has a wavelength in a range between 400 nm and 800 nm; and
- the one or more photosensitizers exhibit antimicrobial efficacy upon activation by electromagnetic radiation;
wherein the surface to be treated is part of clean room clothing and the method is performed after having put on the clean room clothing to be treated, wherein the surface is exposed to electromagnetic radiation for a period of time being less than 5 min and with a radiation dosage between 1 J/cm² and 200 J/cm².

The term "controlling microbial growth", as used herein, denotes any activity for completely inhibiting or at least reducing the growth of microorganisms such as bacteria, archaea, yeasts, fungi, viruses, and the like, in a given environment. In particular, the term relates to biologically controlling microbial growth of said microorganisms, that is, by employing biological means as "growth inhibitors or growth reducing agents." The term "inhibiting", as used herein, is to be understood as not only to include the prevention of further growth of but also to killing of any given microorganisms. The term "reducing", as used herein, denotes any decrease in an microorganism's growth (or growth rate), for example, a decrease of at least 5%, 10%, 15%, 20%, 25%, 30%, 40%, 50%, 60%, 70%, 80%, 85%, 90% or 95% as compared to control conditions (i.e. in the absence of any antimicrobial agents as defined herein). The growth rate may be determined, for example, by simply counting the respective cell numbers (i.e. colony forming units; cfu) after incubation in the presence or absence of any antimicrobial agents, respectively.

The term "surface to be treated", as used herein, denotes any surface that can be colonized by microbial organisms (i.e. contaminants) and to which the one or more photosensitizers of the present invention can be applied.

The surface to be treated is part of clean room clothing.

The term "clean room", as used herein, denotes an area in which quality of air, temperature, humidity, and particle concentration are usually regulated and monitored in order to protect sensitive equipment from contamination. Air in a clean room may be repeatedly filtered to remove dust particles, germs and/ other impurities. Hence, a clean room may be denoted as a room in which the concentration of air-born particulate matter may be controlled at specific limit to facilitate the manufacture of sterile and highly purified products. In pharmaceutical and medical technology, such particles may to be controlled and monitored and germs may be eliminated which might deteriorate the sterile properties of such a room.

The term "clean room lock", as used herein, denotes a device or chamber permitting the passage of people (and optionally objects) between a clean room ("clean side") and a surrounding ("unclean side"). Such a lock may comprise a small chamber with for instance two airtight doors in series which (in the absence of an emergency case) do not open simultaneously. Hence, a clean room lock can be used to allow passage of persons and objects between a clean environment and a less clean environment. Therefore, within such a lock, cleaning procedures may be performed.

The term "clean room clothing", as used herein, refers to clean room clothing, that is, the protective clothing put on before entering a clean room. Within the present invention, the term "clean room clothing" denotes any type of garment such as, e.g., gloves, face masks, bouffants, surgical caps, head covers, hairnets, full cover hoods, socks, shoe covers, sticky mats, sheets, undergarments, coveralls, jackets, shirts, pants, frocks, isolation gowns, and coats. The design of such protective clothing including materials of fabrication as well as functional properties is well known in the art. In a specific embodiment, the clean room clothing used is provided with one or more antimicrobial agents of inorganic origin, for example gold and/or silver fibers incorporated in the fabrication materials.

Usually, clean room clothing is made of one or more textile fabrics. The term "textile fabric" (or "fabric"), as used herein, particularly denotes any flexible material which can be used as a basis for a garment or a suit. For example, such a fabric may comprise a textile material which, in turn, may comprise a network of natural or artificial fibers such as thread or yarn. A textile fabric may be made by weaving or felting or knitting or crocheting natural or synthetic fibers. Many such fibers are well known in the art and commercially available from numerous suppliers (cf., e.g., Gail, L. and Hortig, H.P. (2004), *supra*).

In particularly preferred embodiments of the method according to the invention, the surface to be treated is a textile fabric, and particularly a textile fabric comprising fibers being selected from the group consisting of polyester fibers, cellulose acetate fibers, and combinations (i.e. blended fabrics) thereof.

Both polyester fibers and cellulose acetate fibers are well known in the art. Polyester fibers are composed of polymers having functional ester groups in their backbone. Although there are many polyester fibers known in the art, as a specific material the term most commonly refers to polyethylene terephthalate (PET) fibers. Typically, polyester fibers contain reactive groups, such as hydroxyl (OH)-groups or carboxyl (COOH)-groups. Cellulose acetate fibers represent thermoplastic fibers prepared by reacting cellulose with acetic acid. Both types of fibers can be purchased from various manufacturers.

The term "photosensitizer", as used herein, refers to any compounds that can be activated by light or, more generally, by radiation and that exhibit, upon such activation, antimicrobial activity. Without the intention of being bound by a particular hypothesis, photosensitizers commonly act by producing, upon radiation (light)-induced activation, reactive chemical species (e.g., singlet oxygen) that exert (non-selective) antimicrobial efficacy. Preferably, the one or more photosensitizers do not exhibit any adverse effects on human health.

The term "antimicrobial efficacy", as used herein, denotes an inhibitory (or antagonistic) effect on the growth of microorganisms. In other words, agents that are capable of at least reducing the growth rate (e.g., bacteriostatic agents with respect to controlling the growth of bacteria) as well as agents that cause toxic effects (e.g., bactericide agents killing bacteria) demonstrate antimicrobial efficacy.

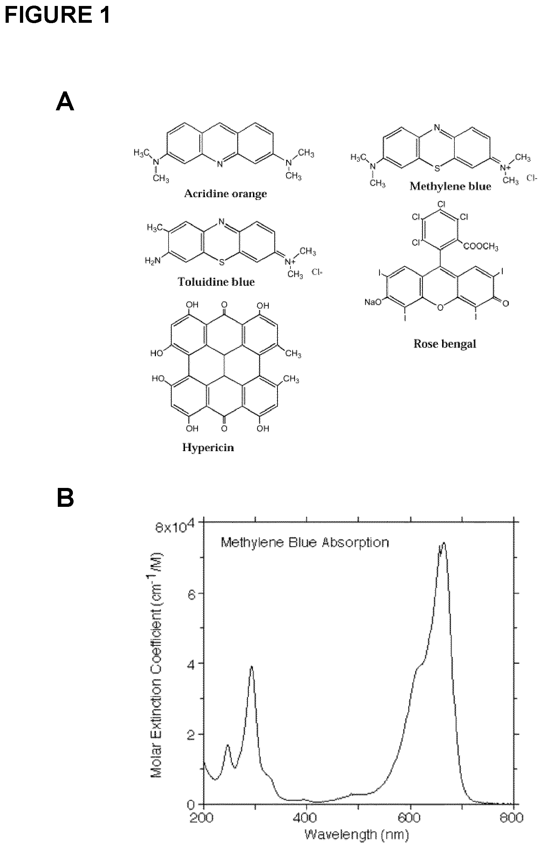

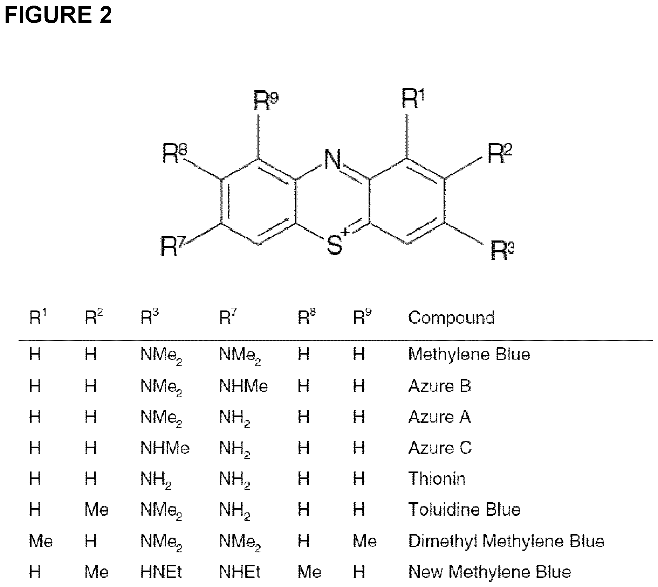

In further preferred embodiments, the one or more photosensitizers are reactive dyes, and particularly preferably reactive dyes being selected from the group consisting of methylene blue (IUPAC name: 3,7-bis(dimethylamino)-phenothiazin-5-ium chloride), methyl methylene blue (IUPAC name: 3,7-bis(dimethylamino)-1-methyldiphenothiazin-5-ium chloride), dimethyl methylene blue (IUPAC name: 3,7-bis(dimethylamino)-1,9-dimethyldiphenothiazin-5-ium chloride), new methylene blue (IUPAC name: 3,7-bis(ethylamino)-2,8-dimethylphenaza-thionium chloride), toluidine blue (IUPAC name: 7-amino-8-methyl-phenothiazin-3-ylidene)-dimethyl-ammonium chloride), Rose Bengal (IUPAC name: 5,6,7-tetrachloro-3',6'-dihydroxy-2',4',5',7-tetraiodo-3*H*-spiro-(iso-benzo-furan-1,9'-xanthen)-3-one), acridine orange (IUPAC name: N,N,*N'*,*N'*-tetramethyl-acridin-3,6-diamin), hypericin (IUPAC name: 1,3,4,6,8,13-hexa-hydroxy-10,11-dimethylphenanthro-[1,10,9,8-opqra]-perylen-7,14-dion), and combinations thereof. The chemical structures of these reactive dyes are shown in Figs. 1A and 2. All of them are well known in the art and commercially available by numerous suppliers.

In some embodiments, the one or more photosensitizers are selected from the group consisting of the above-referenced eight dyes as well as one or more of the dyes being selected from the group consisting of azure A (IUPAC name: N',N'-dimethyl-phenothiazin-5-ium-3,7-diamine chloride), azure B (IUPAC name: 3-methylamino-7-dimethylamino-phenothiazin-5-ium chloride), azure C (IUPAC name: 7-aminophenothiazin-3-ylidene)-methylazanium chloride), and thionin (IUPAC name: 7-aminophenothiazin-3-ylidene)-azanium chloride) (cf. Fig. 2 for chemical structures). Other dyes to be employed herein include *inter alia* Malachite Green (IUPAC name: 4-[(4-dimethylaminophenyl)phenyl-methyl]-*N*,*N*-dimethylaniline), and phloxine B(IUPAC name: 2',4',5',7'-tetrabromo-4,5,6,7-tetrachloro-fluorescein).

In other specific embodiments, the photosensitizer employed is titanium dioxide (TiO₂) which produces reactive species upon irradiation with ultraviolet (UV) light.

In yet other specific embodiments, the one or more photosensitizers employed in the method of the invention are characterized by its/their yield of reactive chemical species, in particular of singlet oxygen, produced upon radiation (light)-induced activation relative to that produced by the reference compound methylene blue. Typically, the one or more photosensitizers employed produce at least 80% or at least 90% of the yield of reactive chemical species (in particular, singlet oxygen) produced by methylene blue. In preferred embodiments, the one or more photosensitizers produce at least 95% or at least 100%, and particularly preferably at least 105%, at least 110%, at least 115%, at least 120%, at least 125% or at least 130% of the yield of reactive chemical species (in particular, singlet oxygen) produced by methylene blue.

The term "one or more" (photosensitizers), as used herein, denotes that the method of the invention may be performed with a single type of photosensitizer (e.g., methylene blue) or with at least two different types (i.e. species) of photosensitizer, wherein the at least two different types may all belong to the group of reactive dyes (e.g., methylene blue and toluidine blue) or at least one reactive dye is combined with at least one other type of compound (e.g., TiO₂). In some particular embodiments, the method is performed with combinations of two or more types of reactive dyes, particularly any one or more (i.e. any subgroup, e.g., any two, any three, any four, any five, any six, any seven, any eight, and so forth) of the reactive dyes referred to above. In exemplary embodiments, the method is performed with one of the following combinations: (methyl methylene blue and dimethyl methylene blue), (methyl methylene blue and new methylene blue), (dimethyl methylene blue and new methylene blue), (methyl methylene blue, dimethyl methylene blue, and new methylene blue).

On a surface to be treated by the method of the invention one or more copies of the one or more (types of) photosensitizers may be provided. Such surface may comprises per surface area of 1 cm² at least 10, at least 50, at least 100, at least 500, at least 1000, at least 5000, at least 10000 or even more copies of the one or more photosensitizers.

The choice of the one or more photosensitizers to be employed for a given scenario may depend *inter alia* on the extent of microbial growth (i.e. the quantity) present on or supposed to be present on the surface to be treated as well as on the nature (i.e. the quality) of the contaminants present (for example, the presence of pathogenic contaminants and/or the presence of multidrug-resistant microbial organisms). The skilled person is also well aware how to select one or more such photosensitizers for a particular application, for example, in order to optimize the radiation (i.e. wavelength, intensity) to be employed by choosing one or more particular photosensitizers, e.g., with overlapping excitation wavelengths, thus resulting in total in a broader wavelength range that is covered. Furthermore, the use of combinations of photosensitizers may result in synergistic effects with respect to antimicrobial efficacy, for example at least 110% as compared to the additive efficacies of the individual photosensitizers or at least 120% as compared to the additive efficacies of the individual photosensitizers. In some embodiments, the method of the present invention further comprises determining and/or monitoring qualitatively and/or quantitatively the microorganisms colonizing the surface to be treated in order to select one or more photosensitizers to be employed. Numerous methods for achieving this goal are also well known in the art.

The one or more photosensitizers provided on the surface to be treated are arranged on or coupled to the surface in any suitable manner. The one or more photosensitizers are covalently attached to and/or incorporated in the surface to be treated. Preferably, the attachment of the one or more photosensitizers is irreversible. For example, in specific embodiments, the one or more photosensitizers may be attached to or incorporated into the textile fabrication materials of clean room clothing, for example by heat treatment (e.g., at a temperature of about 120°C), heat treatment combined with high pressure (e.g., in an autoclave at 121 °C and 210 kPa), ultrasound treatment (at a lower temperature, e.g., of about 50 °C), and the like. The skilled person is well aware of various suitable approaches for attaching the one or more photosensitizers to textile fabrics.

The term "covalent", as used herein, refers to a form of chemical bonding characterized by the sharing of one or more pairs of electrons between two components, producing a mutual attraction that holds the resultant chemical linkage (i.e. molecule) together. Coupling may either be direct (e.g., via reactive groups on the surface to be treated such as OH- or COOH-groups) or via a linker molecule. Numerous such linkers are known in the art (for example, cationic or anionic polymers, such as, e.g., carrageenans (i.e. linear sulfated polysaccharides extracted from red seaweeds)).

The term "electromagnetic radiation", as used herein, refers to a form of energy exhibiting wave like behavior and having both electric and magnetic field components, which oscillate in phase perpendicular to each other as well as perpendicular to the direction of energy propagation. Electromagnetic radiation is classified according to the frequency of its wave. In order of increasing frequency and decreasing wavelength, electromagnetic radiation includes radio waves, microwaves, infrared radiation, visible light, ultraviolet radiation, X-rays, and gamma rays.

The wavelength of the electromagnetic radiation that is used when performing the method of to the present invention is selected (that is, specifically configured or adapted) depending on the one or more photosensitizers provided on the surface to be treated, that is, the electromagnetic radiation (i.e. light) required for activation of the photosensitizers in order to exert their antimicrobial efficacy. The skilled person is well aware how to select an appropriate wavelength depending on the type of photosensitizer(s) used. The type of electromagnetic radiation source emitting radiation having the appropriate wavelength for a given scenario is selected accordingly.

Suitable electromagnetic radiation source to be used in the present invention include *inter alia* laser, mercury lamps, black light lamps, white light lamps, infrared lamps, and light emitting diodes (LEDs), with the latter one being particularly preferred. All these radiation sources are well established in the art.

Within the present invention, one or more electromagnetic radiation sources may be used, which may be of the same type (e.g., at least two LEDs) or of different types (e.g., at least one LED and at least one laser), again depending on the type of photosensitizer(s) provided on the surface to be treated. The one or more electromagnetic radiation sources are arranged in a manner suitable to emit radiation towards the surface to be treated, for example, an arrangement opposite of the surface to be treated. The distance between the one or more electromagnetic radiation sources and the surface to be treated may be in a range between 1 cm and 10 m or in a range between 5 cm and 5 m, and preferably in a range between 10 cm and 4 m or in a range between 20 cm and 3 m. In particularly preferred embodiments, the distance may be in a range between 30 cm and 2.5 m or in a range between 50 cm and 2 m.

The electromagnetic radiation being emitted from the one or more electromagnetic radiation sources (that is, the wavelength required for activating the one or more photosensitizers provided on the surface to be treated) is typically in the range between 400 nm and 800 nm (or between 460 nm and 780 nm) but may also be in a range between 220 nm and 920 nm or in a range of 260 nm and 860 nm. Preferably, the wavelength emitted from the one or more radiation sources is in a range between 580 nm and 650 nm, and particularly preferably in a range between 610 nm and 640 nm.

In specific embodiments employing different types of photosensitizers that are activatable by electromagnetic radiation of different wavelengths, the surface to be treated may be exposed to the radiation of the different wavelengths either simultaneously or consecutively.

The radiation dosage (dose) to be employed is in a range between 1 J/cm² and 200 J/cm², and preferably in a range between 2 J/cm² and 100 J/cm². In specific embodiments, the radiation dosage is less than 80 J/cm², less than 50 J/cm², less than 30 J/cm², less than 25 J/cm², less than 20 J/cm², or less than 15 J/cm².

It is apparent that the duration of the exposure of the surface to be treated to the electromagnetic radiation depend *inter alia* on the type(s) and concentration(s) (i.e. copy numbers) of the one or more photosensitizers employed (in other words, the intensity of the treatment performed), the type(s) and number(s) of at least some of microorganisms whose growth is to be controlled (should this information be available), the size of the surface area to be treated, and the like.

The skilled person is well aware how to calculate the time period required (i.e. the duration of exposure to radiation) for inhibiting the growth of or, preferably, for killing at least a substantial portion of the microbial contaminant(s). Alternatively, reference data retrieved from the scientific literature may be used. In case of clean room clothing, the duration of the incubation period will also depend on whether the method is performed prior to or after having put on the clothing to be treated. Since the method is performed after having put on the clothing to be treated, the time period is shorter, namely less than 5 min, for the sake of convenience of the personnel concerned. The overall incubation period may last, e.g., 5 s, 10 s, 20 s, 30 s, 45 s, 1 min, 2 min, 5 min, 10 min, 20 min, 30 min, 45 min, 1 h, 1.5 h, 2 h, 3 h, 4 h, or 5 h.

In the method according to the present invention, the surface to be treated is exposed to electromagnetic radiation for a period of time being less than 5 min, and preferably for a period of time being less than 4 min, less than 3 min, less than 2 min or less than 1 min.

In preferred embodiments, an exposure of the surface to be treated to electromagnetic radiation for a period of time being less than 5 min, and particularly for a period of time being less than 3 min or less than 2 min is sufficient to reduce the number of microorganisms colonizing the surface to be treated (for example titers of 10°, 10¹, 10², 10³, 10⁴ 10⁵, 10⁶, 10⁷, 10⁸, 10⁹, and 10¹⁰ cfu/ml by at least 50% or at least 60%, and particularly by at least 70% or at lest 80%, as compared to the untreated control. In particular preferred embodiments, the number of microorganisms colonizing the surface to be treated is reduced by at least 90% or at least 95% as compared to the untreated control (including a complete (i.e. 100%) inhibition of microbial growth).

The method according to the present invention is applicable for controlling growth of any microorganisms (i.e. contaminants) colonizing a surface to be treated including pathogenic microorganisms such as human pathogens or plant pathogens. The term "microorganisms", as used herein, denotes any microscopic organism (i.e. organisms too small to be seen by the naked human eye) including both prokaryotic and eukaryotic organism as well as both single cell-organisms and multi-cellular organisms. Examples of microorganisms include *inter alia* bacteria, archaea (archaebacteria), fungi, yeasts, viruses, and protists (e.g., algae, dinoflagellates, amoebae, *Plasmodium spec*., and *Euglena spec*.).

In specific embodiments, the method according to the invention is for controlling growth of extremo-tolerant microorganisms, that is, microorganisms specifically adapted to rather harsh environmental conditions (e.g., depletion of nutrition factors, environmental stresses, and the like), such as to the conditions occurring in clean room environments. Preferably, the microorganisms are selected from the group of bacteria, fungi, and yeasts. Numerous such extremo-tolerant microorganisms have been characterized so far (see, for example, La Duc, M.T. et al. (2007) Appl. Environ. Microbiol. 73, 2600-2611; Moissl, C. et al (2007) FEMS Microbiol. Ecol. 61, 509-521).

In particular embodiments, the method according to the invention is for controlling growth of any one or more clean room associated microorganisms exemplarily selected from the group consisting of *Bacillus spec*., *Paenibacillus spec*., *Geobacillus spec*., *Oceanobacillus spec*., *Micrococcus spec*., *Staphylococcus spec*., *Exiguobacterium spec*., *Microbacterium spec*., *Kocuria spec*., *Pseudomonas spec*., *Sphingomonas spec*., *Stenotrophomonas spec*., *Verticillium spec*., *Penicillium spec*., *Candida spec*., and *Saccharomyces spec*.. All these genera are well known in the art (cf., for example, Garrity, G.M. (ed.) Bergey's Manual of Systematic Bacteriology, Vol. II - The Proteobacteria (Brenner, D.J. et al., eds.), 2nd ed. (2005), Springer Press, New York). The method may be performed for controlling the growth of any one, any subgroup of any two or more (i.e. any two, any three, any four, any five, and so forth) or all of the 16 microorganisms of the group disclosed herein above.

In further specific embodiments, the method is for controlling microbial growth of one or more human pathogens, that is, microorganisms that cause a disease or medical condition in human beings, for example, such as infections, immune diseases, and the like. Particularly, the method is for controlling microbial growth of one or more multidrug-resistant human pathogens. The term "multidrug resistant", as used herein, refers to a condition, where a pathogen is resistant to a variety of drugs or chemical compounds having an otherwise growth inhibitory and/or killing activity on said pathogens. Such drugs or chemical compounds include *inter alia* antibiotics and cytotoxic agents. Examples of such multidrug-resistant microorganisms include *inter alia* multidrug-resistant *Mycobacterium tuberculosis*, multidrug-resistant *Staphylococcus aureus* (including methicillin-resistant *Staphylococcus aureus*), and multidrug-resistant *Haemophilus influenzae*, multidrug-resistant *Enterococcus faecium* (including vancomycin-resistant *Enterococcus faecium*), and multidrug-resistant *Pseudomonas aeruginosa.*

In further specific embodiments, the method of the invention is for controlling growth of one or more human pathogens that cause nosocomial or community-acquired infections. The term "nosocomial infections", as used herein, denotes any infections that are the result of a treatment in a hospital or other clinical setting. Accordingly, this type of infection is also commonly referred to as "hospital-acquired infections". The infection may be caused by any type of microorganisms, that is, they may be of bacterial, viral or fungal origin. Such nosocomial conditions may affect any part of the body including *inter alia* the respiratory tract, the lung, the urinary tract, the gastrointestinal tract, and the bloodstream. In contrast, the term "community-acquired infections", as used herein, denote any infections that are not the result of a treatment in a hospital or other clinical setting.

In particular embodiments, the method according to the invention is for controlling growth of any one or more human pathogenic microorganisms (which may also cause nosocomial infections) being exemplarily selected from the group consisting of *Staphylococcus aureus, Staphylococcus epidermidis*, *Pseudomonas aeruginosa*, *Haemophilus influenzae*, *Stenotrophomonas maltophilia*, *Acinetobacter baumanii*, *Clostridium difficile*, *Mycobacterium tuberculosis*, and *Legionella pneumophila.* All these organisms are well known in the art (cf., for example, Garrity, G.M. (ed.) Bergey's Manual of Systematic Bacteriology, supra). The method may be performed for controlling the growth of any one, any subgroup of any two or more (i.e. any two, any three, any four, any five, and so forth) or all of the 9 microorganisms of the group disclosed herein above.

In a second aspect, the use of a method as defined herein for the microbial decontamination of a surface to be treated is described. In preferred embodiments, the method is used for the complete decontamination (i.e. the inactivation and/or killing of any microorganisms colonizing the surface to be treated). However, it is also possible to use the method for only interfering with the growth of one or more particular species (e.g., pathogenic organisms) that are supposed to be of critical importance for a given application.

In a third aspect, which no longer forms part of the invention, an arrangement for controlling microbial growth on a surface to be treated is described, the arrangement comprising: a surface provided with one or more photosensitizers being activatable by electromagnetic radiation, one or more electromagnetic radiation sources being arranged to emit electromagnetic radiation towards the surface to be treated wherein the one or more electromagnetic radiation sources emit electromagnetic radiation having a wavelength in a range configured for activating the one or more photosensitizers, wherein the electromagnetic radiation being emitted has a wavelength in a range between 400 nm and 800 nm, and wherein the one or more photosensitizers are capable of exhibiting antimicrobial efficacy upon activation. The components of such arrangement are as defined above in the context of the corresponding method for controlling microbial growth on a surface to be treated.

In preferred embodiments, the electromagnetic radiation being emitted has a wavelength in a range between 580 nm and 650 nm, and particularly in a range between 610 nm and 640 nm.

In preferred embodiments of the arrangement, the surface to be treated is a clean room suit, comprising a fabric, and particularly a fabric comprising fibers being selected from the group consisting of polyester fibers, cellulose acetate fibers, and combinations thereof.

In specific embodiments, the one or more photosensitizers are covalently attached to the surface to be treated.

In further preferred embodiments of the arrangement, the one or more photosensitizers are reactive dyes, and particularly reactive dyes being selected from the group consisting of methylene blue, methyl methylene blue, dimethy methylene blue, new methylene blue, toluidine blue, Rose bengal, acridine orange, hypericin, and combinations thereof. Particularly preferably, the one or more photosensitizers are selected from the group consisting of methyl methylene blue, dimethy methylene blue, new methylene blue, and combinations thereof. However, any of the other photosensitizers referred to herein above may be employed as well.

In further specific embodiments, the arrangement further comprises a clean room lock for cleaning a person transmitting between an interior and an exterior of a clean room, wherein the clean room suit is for clothing a person in the clean room lock.
In particularly preferred embodiments of the arrangement, the one or more electromagnetic radiation sources are light emitting diodes.

In specific embodiments, the light emitting diodes are integrated in one or more walls of the clean room lock.

In a forth aspect, which no longer forms part of the invention, a clean room comprising an arrangement, as defined herein, is described.

Finally, in a fifth aspect, which no longer forms part of the invention, the use of an arrangement, as defined herein, or a clean room, as defined herein, for the microbial decontamination of a surface to be treated is described.

The invention is further described by the figures and the following examples, which are solely for the purpose of illustrating specific embodiments of this invention, and are not to be construed as limiting the scope of the invention in any way.

### EXAMPLES

### Example 1: Material and methods

In the subsequent functional analyses, the following materials were employed:

### Microbial strains:

*Staphylococcus aureus* (*S. aureus*) - gram-positive bacterium
*Stenotrophomonas maltophilia* (*S. maltophilia*) - gram-negative bacterium
*Klebsiella pneumoniae* (*K. pneumoniae*) - gram-negative bacterium
*Candida albicans* (*C. albicans*) - yeast

Typically, 3 µl of a growing cell culture having a titer of 7 x 10⁷ cfu/ml were used in contact assays, whereas 50 µl were employed in plating assays. In some assays, the cell cultures were employed in a dilution of 10⁻¹, 10⁻², 10⁻³, 10⁻⁴, and 10⁻⁵, respectively.

### Agar plates:

The growth medium (nutrient agar) employed contained: 4 g/l peptone, 2.4 g/l beef extract, 12 g/l agar; pH 7.1

### Reactive dyes:

Methylene blue (MB), dimethyl methylene blue (DMMB), new metylene blue (NMB), and toluidine blue (TB) were provided as solutions having a dye concentration of 100 µg/ml. 50 µl of either solution were used for plating assays

### Textile:

The textile fabric employed was a clean room textile, in particular ION-NOSTAT VI.2 (Dastex Reinraumzubehör, Muggensturm, Germany). For contact assays, pieces of the textile fabric having a defined size of 1 cm x 1 cm were employed.

### Radiation sources:

Laser (15 mW; wavelength of 532 nm)
mercury lamp (from microscope "LEICA TCS SPE"; excitation filter: 515-560 nm)
black light lamp (Type 75W / E-27; wavelength of 350-370 nm)
LED (illuminant 38 GU5.3 highpower; wavelength of 620-640 nm; "LED1")
LED (illuminant MR16; wavelength of 620-640 nm; "LED2")
LED (High Power Alustar 3 W 10° LEDxON Rot; "LED")

In contact assays, the distance between the radiation source and the piece of textile fabric was in a range between 10 cm and 60 cm.

### Contact assays:

In brief, the contact assays were performed as follows: Pieces of a textile fabric (ION-NOSTAT VI.2) having a defined size (1 x 1 cm) were immersed for 3 h in an aqueous solution of reactive dye, such as methylene blue (100 µg/ml each). After complete drying of the textile pieces, 3 µl of a growing cell culture were added to the surface of the textile pieces. The pieces were irradiated with appropriate radiation (light) for a given period of time. Then, the textile pieces were contacted with agar plates; and the plates were incubated at 37°C for 24 h.

### Plating assays:

In brief, the plating assays were performed as follows: In a well of a (96 well) microtiter plate 50 µl of a growing cell culture were mixed with aqueous solution of reactive dye, such as methylene blue (100 µg/ml each). The samples were irradiated with appropriate radiation (light) for a given period of time. The samples (100 µl) were plated on agar plates. The plates were incubated at 37°C for 24 h.

### Example 2: Analysis of the antimicrobial efficacy of methylene blue attached to textile fabrics (contact assay).

The contact assay was performed as described in Example 1 with cell cultures of *Staphylococcus aureus*, *Stenotrophomonas maltophilia*, and *Candida albicans*, respectively. Irradiation (illumination) of part of the samples was performed for 3 min with a laser (15 mW) at a wavelength of 532 nm. The results of an exemplary assay are shown in Fig. 3.

Fig.3A shows a control plate after contact sampling of textile pieces without further treatment (i.e. irradiation): *S*. *aureus* (left), *S*. *maltophilia* (right). A lawn of bacteria could be observed in with both bacterial strains. Upon irradiation, no colonies (colony forming units; cfu) could be detected for *S*. *aureus* (Fig. 3B, left), and two colonies for *S*. *maltophilia* (Fig. 3B right).

Thus, the treatment results in a complete (*S. aureus*) or almost complete (*S. maltophilia*) growth inhibition, even though irradiation was performed at a wavelength not corresponding to the absorption maximum of methylene blue.

Fig. 3C depicts a corresponding exemplary analysis for *C*. *albicans.* Shown are (in clockwise direction, starting at top left: the control sample (without any treatment), and the test samples after irradiation for 1 min, 2 min, and 3 min, respectively. Upon irradiation, a significant reduction of microbial growth could be observed.

### Example 3: Analysis of the antimicrobial efficacy of methylene blue (plating assay).

The plating assay was performed as described in Example 1 with cell cultures of *Staphylococcus aureus.* Irradiation (illumination) of part of the samples was performed for 3 min with (i) a mercury lamp at a wavelength of 515-560 nm, and (ii) a black light lamp at a wavelength of 350-370 nm. The results of an exemplary assay are shown in Fig. 4. The cells were plated in a dilution of 10⁻⁵.

Figs. 4A and 4C show the results of a control samples, directly plated without further treatment. Fig. 4B shows a test sample after irradiation for 3 min with a mercury lamp. No colonies could be detected. Fig. 4D depicts the results of a test sample after irradiation for 3 min with a black light lamp. Only 15 colonies could be detected.

In a further exemplary analysis using cell cultures of *S*. *aureus* (dilution 10⁻⁵), *S*. *maltophilia* (dilution 10⁻⁵), and *C. albicans* (dilution 10⁻⁴) the following results (per ml) were obtained:

| **MERCURY LAMP** | * Incubation period two days longer as compared to control. | | |
|---|---|---|---|
| **Microbial strain** | ***S. aureus*** | ***S. maltophilia*** | ***C. albicans*** |
| Control | 2.8 x 10⁸ cfu/ml | 4.9 x 10⁷ cfu/ml | 3.4 x 10⁷ cfu/ml |
| Irradiation for 30 s | 2.1 x 10⁷ cfu/ml | 1.4 x 10⁷ cfu/ml | 2.3 x 10⁷ cfu/ml * |
| Irradiation for 3 min | 1.0 x 10⁵ cfu/ml | 0 cfu/ml | 2.2 x 10⁷ cfu/ml * |

| **BLACK LIGHT LAMP** | * Incubation period two days longer as compared to control. | | |
|---|---|---|---|
| **Microbial strain** | ***S. aureus*** | ***S. maltophilia*** | ***C. albicans*** |
| Control | 1.3 x 10⁸ cfu/ml | 1.1 x 10⁸ cfu/ml | 3.3 x 10⁷ cfu/ml |
| Irradiation for 30 s | 1.2 x 10⁸ cfu/ml | 2.9 x 10⁷ cfu/ml | 2.7 x 10⁷ cfu/ml * |
| Irradiation for 3 min | 1.5 x 10⁷ cfu/ml | 0 cfu/ml | 2.3 x 10⁷ cfu/ml * |

### Example 4: Analysis of the antimicrobial efficacy of methylene blue and toluidine blue (plating assay).

The plating assay was performed as described in Example 1 with cell cultures of *Staphylococcus aureus.* Irradiation (illumination) of part of the samples was performed for 3 min with a mercury lamp at a wavelength of 515-560 nm. The results of an exemplary assay are shown in Fig. 5. Preliminary results indicated synergistic effects when using a combination of methylene blue and toluidine blue (data not shown).

Fig. 5A depicts the results obtained with methylene blue: direct plating of the cells (no incubation with dye, no irradiation; i.e. control conditions) yielded 231 cfu (left). After incubation of the cells with the dye (3-5 min) without irradiation 177 cfu were obtained (middle), demonstrating a slight growth inhibiting activity of methylene blue as such. Upon irradiation for 3 min, only one colony could be detected confirming the results obtained in Examples 2 and 3.

Fig. 5B depicts the results obtained with toluidine blue: direct plating of the cells (no incubation with dye, no irradiation) yielded 280 cfu (left). After incubation of the cells with the dye (3-5 min) without irradiation 89 cfu were obtained (middle). Upon irradiation for 3 min, no colony could be detected at all.

### Example 5: Analysis of the antimicrobial efficacy of methylene blue and toluidine blue (contact assay).

The contact assay was performed as described in Example 1 with cell cultures of *S*. *aureus* (dilution 10⁻⁵) and *S*. *maltophilia* (dilution 10⁻⁵), respectively. In addition, after immersing and complete drying of the textile pieces, part of the textile pieces were "pre-irradiated" before contacting them with the cell cultures. "Pre-irradiation" was performed either once for 3 min or twice for 3 min each, with 90 min between the two treatments.

The agar plates of *S*. *aureus* were incubated for 24 h, and the agar plates of *S*. *maltophilia* for 48 h. The following results were obtained (calculated per ml cell culture):

| **METHYLENE BLUE** | | |
|---|---|---|
| **Microbial strain** | ***S. aureus*** | ***S. maltophilia*** |
| Control (no dye) | 2.3 x 10⁸ cfu/ml | 4.9 x 10⁸ cfu/ml |
| Control (dye, no irrad.) | 1.7 x 10⁸ cfu/ml | 1.1 x 10⁸ cfu/ml |
| Irradiation for 3 min | 0 cfu/ml | 1.0 x 10⁵ cfu/ml |
| 1 x pre-irradiation | 0 cfu/ml | 1.0 x 10⁵ cfu/ml |
| 2 x pre-irradiation | 0 cfu/ml | 1.0 x 10⁵ cfu/ml |

| **TOLUIDINE BLUE** | | |
|---|---|---|
| **Microbial strain** | ***S. aureus*** | ***S. maltophilia*** |
| Control (no dye) | 3.8 x 10⁷ cfu/ml | n.d. |
| Control (dye, no irrad.) | 8.9 x 10⁶ cfu/ml | n.d. |
| Irradiation for 3 min | 0 cfu/ml | n.d. |
| 1 x pre-irradiation | 9.0 x 10⁴ cfu/ml | n.d. |
| 2 x pre-irradiation | 5.0 x 10⁴ cfu/ml | n.d. |

The above results demonstrate that both dyes, upon irradiation by light (i.e. activation), have a strong growth inhibitory (antimicrobial) effect on different microbial strains.

### Example 6: Analysis of the antimicrobial efficacy of methylene blue at a wavelength of 620-640 nm (contact and plating assays).

The respective contact and plating assays were performed as described in Example 1 with cell cultures of *Staphylococcus aureus.* Irradiation (illumination) of part of the samples was performed for 3 min with two different LEDs (LED1 and LED2) at a wavelength of 620-640 nm. The distance between the LEDs and the textile was 30 cm. The results of an exemplary assay are shown in Fig. 6.

Fig. 6A depicts the results of the contact assay. After incubation with dye and addition of cell culture without irradiation a lawn of bacteria could be observed (left; two textile pieces tested). Upon irradiation for 2 min with LED1 (middle; three textile pieces tested) or LED2 (right; three textile pieces tested), a significant reduction of the number of colonies could be observed, respectively.

Fig. 6B depicts the results of the plating assay (dilution 10⁻⁵) cell culture. The control sample (no incubation with dye, no irradiation) resulted in 1.2 x 10⁸ cfu/ml (left). Upon irradiation for 2 min with LED1 (middle) and LED2 (right), 1.2 x 10⁷ cfu/ml and 2.0 x 10⁶ cfu/ml could be observed.

### Example 7: Staining of textile fabrics with dimethyl methylene blue.

Pieces of a textile fabric having a defined size of 1 x 1 cm were stained with 100 mg/l dimethyl methylene blue for 15 min in an autoclave (121 °C, 210 kPa). The pieces were repeatedly (at least three times) washed in distilled water to remove excess dye, and then subjected to an ultrasound treatment for 45 min in order to remove unbound dye not attached to the fabric. An exemplary textile piece that was stained according to the above procedure is depicted in Fig. 7A (shown at the left). An unstained control is shown at the right. It could be observed that the staining is homogenous and uniform.

Subsequently, the stained pieces of textile fabric were analyzed with regard to their resistance to various chemicals. The stained pieces were repeatedly (at least two times) immersed in the following chemicals: 99% (v/v) acetone, 99% (v/v) acetic acid, 1 M sodium hydroxide (NaOH), and 99% (v/v) ethanol (EtOH). The wet pieces were then placed on filter paper, and the extent of dye released ("washed out") from the pieces was determined. The results are shown in Fig. 7B. No dye was apparently released upon two cycles of treatment. Trace amounts of released dye could be detected after a single immersion with acetone, acetic acid, and ethanol, respectively (data not shown). However, it is to be noted that the dye concentration used herein (i.e. 100 mg/l) is extremely high and largely exceeds the dye concentrations occurring in practical applications.

Hence, the staining of textile fabrics with reactive dyes (i.e. photosensitizers) according to the invention (here, exemplarily shown for dimethyl methylene blue) result in a uniform color that is also resistant to chemical treatment.

### Example 8: Analysis of the antimicrobial efficacy of dimethyl methylene blue and new methylene blue (contact assay).

The contact assay was performed as described in Example 1 using an aqueous solution of diemethyl methylene blue (DMMB) and new methylene blue (NMB), respectively (100 µg/ml each) as well as a combination thereof. *Staphylococcus aureus* and *Klebsiella pneumoniae* were employed as test organisms. Irradiation was accomplished by means of a LED (High Power Alustar 3 W 10° LEDxON Rot, from Conrad, Hirschau, Germany) at a wavelength of 623 nm. The distance between the LED and the textile pieces was 50 cm.

The results obtained with *S*. *aureus* are shown in Fig. 8A. The following conditions were textile piece, no incubation with dye, no irradiation (right top); textile piece, incubation with NMB, addition of cell culture (about 10⁴ cfu), irradiation for 1 min (left top); textile piece, incubation with DMMB, addition of cell culture, irradiation for 1 min (left bottom); textile piece, incubation with NMB + DMMB, addition of cell culture, irradiation for 1 min (right bottom). After immersing the textile piece with DMMB no cell growth could be detected. In other words, DMMB shows a very potent antimicrobial activity. In contrast, with NMB only a small reduction of cell growth could be observed as compared to control conditions ("blank"). After irradiation of the textile pieces for 2 min and 5 min, respectively, the antimicrobial activity of NMB increased considerably, resulting in a more than 50% reduction of cell growth (data not shown). Comparative analyses with methylene blue and toluidine blue revealed that a complete growth inhibition could only be obtained after irradiation for at least 5 min. Hence, DMMB is a highly efficient photosensitizer according to the invention. Preliminary results indicated synergistic effects when using a combination of NMB and DMMB (data not shown).

The results obtained with *K. pneumoniae* are shown in Fig. 8B. In general, as a result of a different cell wall composition gram-negative bacteria exhibit higher resistance towards reactive chemical compounds than gram-positive bacteria. However, a significant inhibition of cell growth could also be detected for *K. pneumoniae* when using DMMB as photosensitizer. The following conditions were used: textile piece, incubation with DMMB, addition of cell culture (about 10⁴ cfu), irradiation for 2 min (top), 5 min (middle), and 10 min (bottom), respectively. In each experiment, three different dilutions of the cell cultures (10⁻², 10³, and 10⁻⁴) were analyzed. After 5 min of irradiation a noticeable reduction of cell numbers could be observed, with further increase after an irradiation period of 10 min.

## Claims

1. Method for controlling microbial growth on a surface to be treated, comprising: exposing the surface to be treated to electromagnetic radiation, the electromagnetic radiation being emitted from one or more electromagnetic radiation sources towards the surface, wherein:
- one or more photosensitizers being activatable by electromagnetic radiation are covalently attached to the surface to be treated;
- wherein the electromagnetic radiation being emitted has a wavelength in a range between 400 nm and 800 nm; and
- the one or more photosensitizers exhibit antimicrobial efficacy upon activation by electromagnetic radiation;
wherein the surface to be treated is part of clean room clothing and the method is performed after having put on the clean room clothing to be treated, **characterized in that** the surface is exposed to electromagnetic radiation for a period of time being less than 5 min and with a radiation dosage between 1 J/cm² and 200 J/cm².

2. The method of claim 1, **characterized in that** the electromagnetic radiation being emitted has a wavelength in a range between 580 nm and 650 nm, and particularly in a range between 610 nm and 640 nm.

3. The method of claim 1 or 2, **characterized in that** the clean room clothing is a textile fabric comprising fibers being selected from the group consisting of polyester fibers, cellulose acetate fibers, and combinations thereof.

4. The method of any one of claims 1 to 3, **characterized in that** the surface is exposed to electromagnetic radiation for a period of time being less than less than 3 min, particularly less than 1 min.

5. The method of any one of claims 1 to 4, **characterized in that** the one or more electromagnetic radiation sources are light emitting diodes.

6. The method of any one of claims 1 to 5, **characterized in that** the method is for controlling growth of any one or more clean room associated microorganisms being selected from the group consisting of *Bacillus spec., Paenibacillus spec., Geobacillus spec., Oceanobacillus spec., Micrococcus spec., Staphylococcus spec., Exiguobacterium spec., Microbacterium spec., Kocuria spec., Pseudomonas spec., Sphingomonas spec., Stenotrophomonas spec., Verticillium spec., Penicillium spec., Candida spec.,* and *Saccharomyces spec..*

## Patentansprüche

1. Verfahren zur Steuerung von mikrobiellem Wachstum auf einer zu behandelnden Oberfläche, wobei das Verfahren Folgendes umfasst:
Aussetzen der zu behandelnden Oberfläche einer elektromagnetischen Strahlung, wobei die elektromagnetische Strahlung von einer oder mehreren elektromagnetischen Strahlungsquellen zur Oberfläche emittiert wird, wobei:
- ein oder mehrere Photosensitizer, die durch elektromagnetische Strahlung aktivierbar sind, kovalent an die zu behandelnde Oberfläche gebunden sind;
- wobei die emittierte elektromagnetische Strahlung eine Wellenlänge in einem Bereich zwischen 400 nm und 800 nm hat; und
- die einen oder mehreren Photosensitizer eine antimikrobielle Wirksamkeit
bei Aktivierung durch elektromagnetische Strahlung zeigen;
wobei die zu behandelnde Oberfläche Teil von Reinraumkleidung ist und das
Verfahren durchgeführt wird, nachdem die zu behandelnde Reinraumkleidung angezogen wurde,
**dadurch gekennzeichnet dass**
die Oberfläche elektromagnetischer Strahlung über einen Zeitraum von weniger als 5 min und mit einer Strahlungsdosis zwischen 1 J/cm² und 200 J/cm² ausgesetzt wird.

2. Verfahren nach Anspruch 1, **dadurch gekennzeichnet dass** die emittierte elektromagnetische Strahlung eine Wellenlänge in einem Bereich zwischen 580 nm und 650 nm, und insbesondere in einem Bereich zwischen 610 nm und 640 nm, hat.

3. Verfahren nach Anspruch 1 oder 2, **dadurch gekennzeichnet dass** die Reinraumkleidung ein textiles Flächengebilde ist, das Fasern umfasst, die aus der Gruppe, bestehend aus Polyesterfasern, Celluloseacetatfasern und Kombinationen davon, ausgewählt sind.

4. Verfahren nach einem der Ansprüche 1 bis 3, **dadurch gekennzeichnet dass** die Oberfläche elektromagnetischer Strahlung über einen Zeitraum von weniger als 3 min, insbesondere weniger als 1 min, ausgesetzt wird.

5. Verfahren nach einem der Ansprüche 1 bis 4, **dadurch gekennzeichnet dass** die eine oder mehreren elektromagnetischen Strahlungsquellen Licht emittierende Dioden sind.

6. Verfahren nach einem der Ansprüche 1 bis 5, **dadurch gekennzeichnet dass** das Verfahren zur Steuerung des Wachstums von einem oder mehreren mit einem Reinraum in Verbindung stehenden Mikroorganismen ist, die aus der Gruppe, bestehend aus *Bacillus spec., Paenibacillus spec., Geobacillus spec., Oceanobacillus spec., Micrococcus spec., Staphylococcus spec., Exiguobacterium spec., Microbacterium spec., Kocuria spec., Pseudomonas spec., Sphingomonas spec., Stenotrophomonas spec., Verticillium spec., Penicillium spec., Candida spec.* und *Saccharomyces spec.,* ausgewählt sind.

## Revendications

1. Procédé de contrôle de la croissance microbienne sur une surface à traiter, comprenant : l'exposition de la surface à traiter à un rayonnement électromagnétique, le rayonnement électromagnétique étant émis d'une ou plusieurs sources de rayonnement électromagnétique vers la surface, dans lequel :
- un ou plusieurs photosensibilisateurs qui sont activables par rayonnement électromagnétique sont fixés de façon covalente à la surface à traiter ;
- dans lequel le rayonnement électromagnétique qui est émis a une longueur d'onde dans une plage entre 400 nm et 800 nm ; et
- les un ou plusieurs photosensibilisateurs présentent une efficacité antimicrobienne lorsqu'ils sont activés par un rayonnement électromagnétique ;
dans lequel la surface à traiter est une partie d'un vêtement de salle blanche et le procédé est réalisé après avoir porté le vêtement de salle blanche à traiter,
**caractérisé en ce que** la surface est exposée à un rayonnement électromagnétique pendant une durée de moins de 5 min et avec une dose de rayonnement entre 1 J/cm² et 200 J/cm².

2. Procédé selon la revendication 1, **caractérisé en ce que** le rayonnement électromagnétique qui est émis a une longueur d'onde dans une plage entre 580 nm et 650 nm, et en particulier dans une plage entre 610 nm et 640 nm.

3. Procédé selon la revendication 1 ou 2, **caractérisé en ce que** le vêtement de salle blanche est un tissu textile comprenant des fibres choisies dans le groupe constitué des fibres de polyester, des fibres d'acétate de cellulose, et de leurs combinaisons.

4. Procédé selon l'une quelconque des revendications 1 à 3, **caractérisé en ce que** la surface est exposée à un rayonnement électromagnétique pendant une durée de moins de 3 min, en particulier de moins de 1 min.

5. Procédé selon l'une quelconque des revendications 1 à 4, **caractérisé en ce que** les une ou plusieurs sources de rayonnement électromagnétique sont des diodes électroluminescentes.

6. Procédé selon l'une quelconque des revendications 1 à 5, **caractérisé en ce que** le procédé est destiné au contrôle de la croissance d'un ou plusieurs quelconques micro-organismes associés à une salle blanche qui sont choisis dans le groupe constitué de *Bacillus spec., Paenibacillus spec., Geobacillus spec., Oceanobacillus spec., Micrococcus spec., Staphylococcus spec., Exiguobacterium spec., Microbacterium spec., Kocuria spec., Pseudomonas spec., Sphingomonas spec., Stenotrophomonas spec., Verticillium spec., Pénicillium spec., Candida spec.,* et *Saccharomyces spec.*
